# EUROPÄISCHE PATENTSCHRIFT

(11) **EP 2 545 541 B1**
(45) Veröffentlichungstag und Bekanntmachung des Hinweises auf die Patenterteilung: **21.09.2016**
(21) Anmeldenummer: 10718433.5
(22) Anmeldetag: 11.03.2010
(51) Int. Cl.: G09B 23/28, A61B 5/055

(54) **MRI-TRAININGSEINRICHTUNG**
MRI TRAINING DEVICE
SYSTÈME D'APPRENTISSAGE IRM

(43) Veröffentlichungstag der Anmeldung: 16.01.2013
(73) Patentinhaber: Noras, Hubert, 97082 Würzburg (DE)
(72) Erfinder: Noras, Hubert, 97082 Würzburg (DE)
(74) Vertreter: Pöhner, Wilfried Anton
(86) Internationale Anmeldenummer: PCT/DE2010/000262
(87) Internationale Veröffentlichungsnummer: WO 2011/110139

(56) Entgegenhaltungen:
- EP-A1- 0 640 842
- WO-A2-2008/021720
- US-A1- 2002 156 365
- US-A1- 2005 080 333
- US-A1- 2007 016 003

## Beschreibung

Die Erfindung bezieht sich auf eine MRI-Trainings- und Justage-Einrichtung für das Positionieren der Spitze eines medizinischen Werkzeugs in lebendem Gewebe sowie ein Verfahren zu dessen Verwendung.

Als bildgebende Verfahren für eine nicht verletzende Untersuchung des Körperinneren von Lebewesen sind seit etwa einem Jahrhundert Röntgengeräte im Prinzip bekannt. Wegen ihrer radioaktiven Belastung wurden vor etwa drei Jahrzehnten ultraschallgestützte, bildgebende Verfahren eingeführt. Das jüngste Verfahren ist die Magnet-Resonanz-Tomographie, auch Kernspintomographie, Nuklear-Magnet-Resonanz (NMR)-Tomographie oder Magnetic-Resonance-Imaging (MRI) genannt. Ebenso wie das Ultraschall-Verfahren schädigt es den lebenden Organismus nicht, kann aber im Gegensatz dazu auch Knochen abbilden und ist daher auch für vollständige und großflächige Schnittbilder des gesamten Körpers geeignet.

Der zu untersuchende Körper wird in ein starkes und homogenes statisches Magnetfeld - das Hauptmagnetfeld - hinein gefahren, wodurch sich im Körper der Kernspin von Atomkernen, insbesondere von an Wasser gebundenen Wasserstoffatomkernen (Protonen) ausrichtet. Hochfrequente Anregungspulse regen diese Kerne zu einer "Präzessionsbewegung" an. Nach dem Abklingen eines solchen Hochfrequenzanregungspulses "präzedieren" die Atomkerne mit der sog. "Larmorfrequenz" in Abhängigkeit von der Stärke des Hauptmagnetfeldes und schwingen nach einer gewebsabhängigen Relaxationszeit wieder in die Vorzugsrichtung ein, die durch das Hauptmagnetfeld vorgegeben wird.

Für jede Körperschicht kann durch rechnerische und messtechnische Analyse der integralen, hochfrequenten Kernsignale aus der Verteilung der räumlichen Spindichte in Verbindung mit den Relaxationszeiten ein Bild generiert werden. Das infolge der Präzessionsbewegung nachweisbare Kernresonanzsignal kann durch die Anwendung linearer Feldgradienten dem Ort seiner Entstehung zugeordnet werden. Dazu werden dem Hauptmagnetfeld entsprechende Gradientenfelder überlagert und so gesteuert, dass nur in einer abzubildenden Schicht die Kerne angeregt werden. Sowohl zur HF-Anregung der Kernspins als auch zur Detektion der Antwortsignale der Kerne ist eine HF-Spule erforderlich.

So kann z.B. um eine weibliche Brust, die in Bauchlage der Patientin nach unten hängt, eine Brust-Spule herumgelegt werden. Dann können Anomalien im Gewebe, wie z.B. Krebsgeschwulste, bereits dann erkannt werden, wenn sie noch sehr klein sind.

Mit dem MRI-Gerät kann diagnostiziert werden, ob Anomalien vorhanden sind und an welcher Position sie sich befinden. Das MRI-Gerät ermöglicht diese Diagnose ohne jede Verletzung des Patienten - was ein sehr wichtiger Vorzug ist. Wenn jedoch Anomalien erkannt worden sind, so ist ein physischer Zugang dorthin erforderlich.

Um zu beurteilen, ob es Krebsgeschwulste oder andere Anomalien sind, ist es eine sehr sichere Methode, winzige Gewebeprobe zu entnehmen (Biopsie), die dann labortechnisch untersucht werden.

Oder es sollen Medikamente an den Ort der Anomalie möglichst exakt eingespritzt werden, es muss also auch dafür die Spitze eines medizinischen Werkzeuges - wie z.B. einer Hohlnadel - an den Ort der detektierten Anomalie gebracht werden.

Das Einstechen dieses Werkzeuges ist jedoch innerhalb des MRI-Gerätes so gut wie unmöglich: Zum einen erschwert die große Spule des Hauptmagnetfeldes den mechanischen Zugang und zum anderen können wegen des besonders starken Magnetfeldes keine magnetisierbaren Werkstoffe, wie z.B. Stahl verwendet werden.

Deshalb muss der Patient für die Untersuchung und/oder Behandlung aus dem MRI-Gerät herausgefahren werden und das medizinische Werkzeug anhand der soeben ermittelten Positionsdaten positioniert werden. Um diesen Vorgang zu trainieren und auch um die dafür benutzte Vorrichtung zu justieren sind sog. "Phantome" bekannt. Das sind im MRI-Bild gar nicht oder kaum sichtbare Gegenstände in denen bestimmte Punkte durch Materie markiert sind, die im MRI-Gerät ganz deutlich sichtbar sind.

Ein solches Phantom beschreibt das Patent DE 10 2005 050 839 B3. Es besteht aus zahlreichen, im MRI sichtbaren, kugelförmigen Elementen, die in einem gleichmäßigen Raster über das gesamte Volumen des Phantoms verteilt sind. Zur Orientierung sind einige wenige dieser im MRI sichtbare Raumpunkte abweichend von den übrigen gestaltet.

Ein derartiges Phantom ist für die Korrektur von nicht ganz korrekt berechneten Positionsdaten des MRI-Gerätes in jedem Punkt des Raumes geeignet. Durch die große Anzahl von im MRI sichtbaren Zielpunkten ist es jedoch praktisch kaum möglich, damit zu trainieren, wie ein medizinisches Werkzeug mit seiner Spitze genau in einen bestimmten Raumpunkt hingeführt werden kann, weil der Weg dorthin von zahlreichen anderen Kugeln im Raum versperrt ist.

Der wesentliche Nachteil dieses Phantoms ist jedoch, dass der Anwender damit alleine gelassen wird, wie er das Phantom im MRI-Gerät anordnen und befestigen soll und wie sichergestellt wird, dass nach dem Herausfahren des Phantoms aus der Hauptmagnetfeldspule das Phantom so genau gegenüber dem medizinischen Werkzeug positioniert wird, dass die im MRI-Verfahren ermittelten Positionswerte des im Phantom zu erreichenden Punktes auch außerhalb des MRI-Gerätes erreicht werden können.

Ebenfalls nicht beschrieben wird, wie das Einbringen des medizinischen Werkzeugs, also z. B. das Einstechen einer Hohlnadel, tatsächlich abläuft.

Ein weiterer Nachteil des beschriebenen Phantoms ist, dass es keine Hilfe für die Planung von Einstichort und Einstichrichtung gibt.

Auf aktuellem Stand der Technik beschreibt die US 2002/015 63 65, Tsekos, ein Gestell, das in ein MRI-Gerät hinein geschoben werden kann und das bewegliche Rahmen enthält, die an weibliche Brüste oder andere zwischen die Platten hängende Extremitäten herangedrückt werden kann. Die Andrückplatten bringen das menschliche Gewebe in eine bestimmte Position. Wenn in einem derart fixierten Gewebe mit Hilfe des MRI-Gerätes z.B. ein Tumor lokalisiert worden ist, dann kann eine Spritze oder ein Werkzeug an diese Koordinaten gebracht werden und dort z. B. ein Heilmittel einspritzen oder eine Probe entnehmen. Um dieses Verfahren zu üben soll anstelle eines Patienten ein Phantom eingesetzt werden, das in einem hölzernen Rahmen gehalten wird.

Ein wesentlicher Nachteil dieses Prinzips ist es jedoch, dass für das prinzipielle Üben des Einbringens eines Werkzeuges, z. B. des Ein stechens der Nadel, stets das gesamte MRI-Gerät benötigt wird. Durch die hohen Investitionskosten für ein MRI-Gerät wird eine derartige Übungsstunde sehr teuer und blockiert zudem die Benutzung des MRI-Gerätes für Notfälle, die in dieser Zeit eintreffen.

Auf diesem Hintergrund hat sich die Erfindung die Aufgabe gestellt, eine Trainings- und Justage-Einrichtung für das Positionieren der Spitze eines medizinischen Werkzeuges in lebendem Gewebe zu schaffen, bei der ein im MRI-Gerät erfasster und vermessener Raumpunkt an einem Phantom außerhalb eines MRI-Gerätes exakt und auf einer möglichst kurzen Strecke mit der Spitze eines medizinischen Werkzeugs erreicht werden kann.

Mit der Trainings-Einrichtung wird vor allem die korrekte Übertragung der messtechnisch im MRI-Gerät an einem virtuellen Bild ermittelten Positionsdaten auf ein real existierendes, medizinisches Werkzeug geübt. Zusätzlich kann der Mediziner auch trainieren, wie er sein Werkzeug schnell und ohne Irrtümer bei der Positionierung, also mit der geringstmöglichen Belastung des Patienten, in das Gewebe einführt.

Als Lösung lehrt die Erfindung ein Verfahren gemäß den Merkmalen des Anspruchs 1 sowie eine Vorrichtung zur Durchführung des Verfahrens (Anspruch 4).

Ein wesentliches Merkmal der Erfindung ist also die Verbindung einer Einrichtung zum mechanischen Fixieren von weichem Körpergewebe mit einem Phantom.

Diese Einrichtung besteht aus einem Gewebeandrückrahmen, der auf Gewebe, wie z.B. eine weibliche Brust angedrückt wird. Dieser Rahmen wird von wenigstens einer Andrückschiene durchkreuzt. Bevorzugter Weise wird die Öffnung im Gewebeandrückrahmen von einer gitterartigen Struktur aus mehreren Andrückschienen gleichmäßig ausgefüllt. Der gesamte Gewebeandrückrahmen ist an wenigstens einem Befestigungsblock montiert, der gegenüber der Grundplatte linear verschiebbar ist.

Zur Übung des Einbringens eines medizinischen Werkzeuges an einen bestimmten Raumpunkt - z.B. des Einstechens mit einer Nadel - kann die Grundplatte mitsamt dem Gewebeandrückrahmen und einem Phantom auch außerhalb eines MRI-Gerätes - z.B. auf einem Tisch - genutzt werden.

Die Funktion des Gewebeandrückrahmens im MRI-Gerät soll am Beispiel einer weiblichen Brust erläutert werden. Dazu wird die Patientin in Bauchlage so hingelegt, dass ihre Brüste durch je eine Öffnung hindurchhängen, die von je einer MRI-Spule umgeben ist. Unterhalb dieser MRI-Spulen und in einem Abstand dazu wird die Grundplatte befestigt, die den Gewebeandrückrahmen trägt. Er kann - z.B. durch einen Spindelantrieb - gegenüber der Grundplatte linear verschoben werden und zwar so weit, bis er auf die nach unten hängende Brust auftrifft und sie komprimiert. Sinnvollerweise ist dazu auf der dem Gewebeandrückrahmen gegenüberliegenden Seite der Brust ein entsprechendes Gegenstück angeordnet, das entweder ortsfest ist oder sogar gegenläufig zum Gewebeandrückrahmen verschoben wird, sodass der Gewebeandrückrahmen und sein Gegenstück die Brust ähnlich wie ein Schraubstock einklemmen.

Dadurch wird die Brust in eine definierte Form gebracht, in der eventuelle Anomalien des Gewebes in eine bestimmte Position gebracht werden. Auch durch die Atmung des Patienten wird die Brust nicht gegenüber der Einrichtung "verschoben". Bei einem Verbleib des Gerätes an der Brust ist diese Position reproduzierbar wieder erreichbar.

Das besondere des Gewebeandrückrahmens ist, dass er auf fast der gesamten Fläche den Zutritt des medizinischen Werkzeuges in das lebende Gewebe ermöglicht. Dazu haben die Andrückschienen in der Regel ein sehr schmales Profil, das senkrecht zur Oberfläche des lebenden Gewebes ausgerichtet ist und auf dieses Weise nur einen sehr geringen Anteil der Fläche blockiert.

In der Praxis wird die Patientin - wie erwähnt - auf dem Untersuchungstisch eines MRI-Gerätes in Bauchlage platziert, wobei ihre Brüste durch je eine MRI-Spule nach unten hängen. Noch außerhalb der Hauptfeldspule werden die Brüste durch lineares Verschieben des Gewebeandrückrahmens etwas komprimiert und dadurch in eine definierte Position gebracht. Dann wird im nächsten Schritt die Patientin mitsamt dem Gewebeandrückrahmen und dessen Befestigungseinrichtung in das MRI-Gerät hineingefahren und die Brust untersucht.

Wenn dabei Gewebeanomalien entdeckt werden, so wird ihre Position vom MRI-Gerät ermittelt und dem untersuchenden Mediziner zur Kenntnis gebracht. Im nächsten Schritt wird die Patientin wieder aus dem MRI-Gerät herausgefahren, sodass der Mediziner die immer noch zwischen dem Gewebeandrückrahmen und dessen Gegenstück komprimierte Brust erreichen kann.

In dieser Position kann er sein medizinisches Werkzeug in die Brust einführen, sodass die Spitze des Werkzeuges die Gewebeanomalie erreicht. Dort kann er z.B. eine Gewebeprobe entnehmen (Biopsie) oder eine Markierung hinterlassen, sodass die Gewebeanomalie für spätere Behandlungen, wie z.B. einer Operation eindeutig identifiziert werden kann. Eine Möglichkeit der Therapie ist es, durch das medizinische Werkzeug hindurch Wirkstoffe in den Bereich der Anomalie einzubringen.

Um diesem Ablauf für die Patientin so schmerzarm und so kurz wie nur irgend möglich zu gestalten, kann er mit der erfindungsgemäßen MRI-Trainigs- und Justage-Einrichtung gründlich geübt werden und die Vorrichtungen für das Einbringen der Nadel kann justiert werden.

Dazu wird anstelle des Gewebeandrückrahmens oder hinter den Gewebeandrückrahmen dein sog. "Phantom" in eine bestimmte Position fest montiert. Innerhalb des Phantoms sind Ziel körper enthalten, die im MRI bildgebend sind. Die übrigen Bestandteile des Phantoms, wie z.B. seine Wände und seine Füllung sind im MRI gar nicht oder nur geringfügig sichtbar. Dann können im MRI-Gerät die Positionen der Zielkörper vermessen werden und deren Raumkoordinaten bekannt gegeben werden. Im nächsten Schritt wird das Phantom mitsamt seiner Befestigung und der Grundplatte in genau der Position in der es auch vermessen worden ist, aus dem MRI-Gerät herausgefahren und der handelnde Mediziner kann im nächsten Schritt sein Werkzeug in das Phantom einbringen.

Dabei übt er es, die vorhandenen Zielkörper genau zu erreichen und dafür nur die vom MRI-Gerät zur Verfügung gestellten Positionsdaten zu nutzen. Wenn das Gehäuse des Phantoms und die Füllung des Phantoms transparent sind, so kann er durch Inaugenscheinnahme das Ergebnis seiner Übungstätigkeit kontrollieren.

Wenn sich bei diesen Übungen stets gleiche, reproduzierbare Abweichungen ergeben, so kann daraus ein Korrekturwert abgeleitet werden, mit dem die Führung des medizinischen Werkzeuges beaufschlagt werden muss.

Um nicht nur das korrekte "Zielen" mit dem Werkzeug zu üben, sondern um dem Mediziner auch ein Gefühl für das Einbringen des Werkzeuges in das menschliche Gewebe zu verschaffen, schlägt die Erfindung als Ausführungsform vor, dass das Phantom ein Behälter ist, dessen zur Öffnung des Gewebeandrückrahmens weisende Fläche aus einer dünnen, elastischen und hautartigen Membran besteht, die von dem medizinischen Werkzeug durchdringbar ist. Damit wird simuliert, dass die Haut in der Regel dem Werkzeug einen höheren Widerstand entgegengesetzt wie das innere Gewebe.

Deshalb wird sich die Membran bei der ersten Berührung durch das medizinische Werkzeug nicht sofort öffnen, sondern ein wenig zurückweichen. Erst wenn der Druck des Werkzeuges so groß wird, dass die Elastizität der Haut überschritten ist, reißt sie etwas ein, wodurch sich ein Einlass für das medizinische Werkzeug auftut, durch welchen hindurch es in das Gewebe eindringen kann.

Damit auch innerhalb des Phantoms das Verhalten des medizinischen Werkzeuges beim Eindringen in des Gewebe möglichst genau nachgebildet wird, schlägt die Erfindung vor, dass es mit einen niedrig viskosen, gallertartigen Gel oder einer anderen dem menschlichen Gewebe in seinen mechanischen Eigenschaften und seinem Bild nahekommendem Material befüllt ist. Der größte Anteil dieses Gels - etwa 80-93% - besteht aus Wasser, Konservierungsstoffen, Quervernetzern und Antibiotika. Die übrigen 7-20% sind Substantatine (??). Eine andere Rezeptur wäre 0,5-3,0 g NiS04 X 6H2O, und 3,0-7,0 NaCl auf ein Liter destilliertes Wasser.

Als eine sinnvolle, mechanische Ausführungsform schlägt die Erfindung vor, dass auf der Grundplatte zwei Befestigungsblöcke im Abstand zueinander angeordnet sind, die mittels je einer Gewindespindel in Richtung des lebenden Gewebes verschiebbar sind und an denen das Phantom und/oder der Gewebeandrückrahmen befestigt werden können.

Als eine Möglichkeit zur Befestigung schlägt die Erfindung T-förmige Nuten vor, in welche entsprechend komplementär geformte Gegenstücke eingreifen, die an das Phantom und an den Gewebeandrückrahmen angeformt sind. Alternativ sind auch Schwalbenschwanzverbindungen, Zapfen und Bohrungen, Rastverbindungen oder Verschraubungen denkbar.

Eine sehr sinnvolle Kombination für das Üben und Justieren der Einrichtung ist es, wenn an den Befestigungsblöcken sowohl der Gewebeandrückrahmen als auch das Phantom befestigt sind. Dann findet der Nutzer genau die gleichen Randbedingungen vor, wie bei der Anwendung an einem Patienten.

Die Kombination aus Gewebeandrückrahmen und Phantom ist insbesondere dann interessant, wenn das Entnahmewerkzeug manuell eingeführt werden soll und als Orientierung nur das jeweilige Feld in der rasterförmig mit horizontalen und vertikalen Andrückschienen ausgefüllten Öffnung des Geweberahmens sowie der Winkel des Werkzeuges in horizontaler und in vertikaler Richtung dienen soll.

Aber auch dann, wenn das medizinische Werkzeug mit einer mechanischen Werkzeughalterung geführt wird, ist es sinnvoll, zusätzlich zum Phantom auch den Gewebeandrückrahmen in der MRI-Trainingseinrichtung zu montieren. Dann kann geprüft werden, ob tatsächlich keine Andrückschienen vom medizinischen Werkzeug getroffen werden.

Damit der Gewebeandrückrahmen sowohl für Training und Justage als auch am Patienten einsetzbar ist, wird vorgeschlagen, dass er von dem Phantom getrennt werden kann.

Damit das Phantom alternativ direkt mit den Befestigungsblöcken verbunden werden kann, ist es sinnvoll, an seine Vorderseite die gleichen Befestigungseinrichtungen anzuformen, die auch an der Vorderseite des Gewebeandrückrahmens vorhanden sind. In diesem Fall muss der Gewebeandrückrahmen an seiner Rückseite ebenfalls T-förmige Nuten aufweisen, die das dazu komplementäre Gegenstück des Phantoms aufnehmen.

Alternativ zu einer manuellen Führung des medizinischen Werkzeuges schlägt die Erfindung eine mechanische Werkzeughalterung vor. Eine dafür geeignete Vorrichtung wird in der Patentschrift DE 196 26 286 C5 beschrieben, die hiermit ausdrücklich zu einem Teil dieser Anmeldung erklärt wird.

Im Prinzip ist jedoch auch eine nur in drei Freiheitsgraden verstellbare Halterung für das Werkzeug denkbar. Sinnvoll wäre dafür eine im Bezug auf den Gewebeandrückrahmen horizontale und eine darauf aufbauende vertikale Verstellung. Beide Verstellungen könnten linear ausgeführt werden. Der dritte Freiheitsgrad wäre die Eindringtiefe des medizinischen Werkzeugs in das lebende Gewebe. Mit einer solchen Verstellung in nur drei Freiheitsgraden sind alle Punkte des Arbeitsraumes erreichbar.

Ein Problem könnte jedoch dann entstehen, wenn das medizinische Werkzeug genau auf eine Andrückschiene trifft. Bei nur geringer Anforderung an die Genauigkeit könnte versucht werden durch die Elastizität des medizinischen Werkzeuges und die Elastizität der Andrückschiene das medizinische Werkzeug doch noch an der Andrückschiene vorbei in das lebende Gewebe zu schieben. Allerdings würde die Positioniergenauigkeit darunter spürbar leiden.

Um diesen Nachteil zu vermeiden und auch um eine möglichst kurze Wegstrecke von der Außenhaut des menschlichen Gewebes bis zum Zielpunkt zu erreichen, schlägt die Erfindung einen weiteren Freiheitsgrad vor. Z.B. könnte durch wenigstens eine Schwenkachse oder besser noch mit zwei Schwenkachsen gewählt werden, aus welcher Richtung im Raum das medizinische Werkzeug in das lebende Gewebe hineingedrückt werden soll.

Als eine weitere Variante für einen möglichst kurzen Weg des medizinischen Werkzeuges von der Haut bis zum Zielpunkt im menschlichen Gewebe empfiehlt die Erfindung eine zusätzliche Schwenkachse, deren geometrischer Mittelpunkt auf der Längsachse der MRI-Spule liegt.

Wenn als Anwendungsbeispiel an eine etwa halbkugelförmige weibliche Brust gedacht wird, bei der eine zu untersuchenden Gewebeanomalie recht nahe an der Oberfläche liegt, dann ist es sinnvoll, auch diese große Schwenkachse so weit zu verschwenken, dass das medizinische Werkzeug etwa senkrecht zur Hautoberfläche in das Gewebe hineingeschoben wird.

Als ein sehr interessantes Ausführungsbeispiel schlägt die Erfindung eine Werkzeughalterung aus mehreren orthogonal zueinander ausgerichteten Schlitten und/oder Schwenkvorrichtungen vor. Diese Schlitten und/oder diese Schwenkvorrichtungen können entweder manuell verstellt und durch je eine Klemmschraube fixiert werden. Alternativ können sie mit je einem Motor bewegt werden.

Ihre jeweilige Position kann über eine mechanische Skala in unmittelbarer Nähe der Bahn eines Schlittens abgelesen werden oder über elektronische Positionsistwertgeber erfasst werden, die mit der Bewegungsmechanik verbunden sind. Sie können z.B. in den Antriebsmotor integriert sein oder bei einer Spindel an dem anderen, freien Ende gegenüber dem Antriebsmotor angeflanscht werden.

Die Antriebsmotoren können in einer sehr einfachen Variante durch einfache Tasten aktiviert werden, die jeweils nur einen einzigen Geschwindigkeitsbereich und eine einzige Drehrichtung vorgeben. Komfortabler sind Hebel, die verschiedene Geschwindigkeitsstufen auswählen können bis hin zu einer stufenlosen Vorgabe der Geschwindigkeit, ähnlich wie beim Fahrpedal (Gaspedal) eines Kraftfahrzeuges.

Bei einer derartigen Ansteuerung der Motoren sollten die eventuell vorhandenen Positionsistwertgeber jeweils mit einer Positionsistwertanzeige verbunden werden. Je nach den für diese Positionsistwertanzeigen verwendeten Einheiten kann dadurch sehr schnell der vom MRI-Gerät vorgegebene Positionssollwert mit dem tatsächlich erreichten Positionsistwert verglichen werden.

Eine Alternative ist es, sowohl die Motoren als auch die Positionsistwertgeber elektrisch mit einer elektronischen Steuerung zu verbinden. In einem sehr einfachen Fall werden alle Positionssollwerte für die jeweiligen Antriebsmotoren mit Ausnahme der Eindringtiefe vorgegeben. Im ersten Schritt wird der damit festgelegte Raumpunkt noch außerhalb des Gewebes oder außerhalb des Phantoms angefahren. Erst wenn dieser Punkt erreicht ist und nur noch das Werkzeug in das Gewebe eingetrieben werden muss, wird im zweiten Schritt der jeweilige Antrieb aktiviert, um das Werkzeug genau in die erforderliche Tiefe zu schieben.

Für die Anordnung der Zielkörper im Innenraum des Phantoms schlägt die Erfindung als eine einfache und übersichtliche Anordnung einen Phantomkörper mit mehreren treppenartige Stufen vor. In jede Stufe ist eine Einsenkung eingeformt, die je einen Zielkörper aufnimmt. In dem einfachen Fall einer nicht exakt vermessenen Höhe der verschiedenen Stufen, dienen sie nur zur Halterung je eines Zielkörpers.

Wenn der genaue Abstand der Stufen zueinander bekannt ist, dann kann nach dem erfolgreichen Auffinden eines ersten Zielkörpers die relative Genauigkeit der Positionsistwerterfassung des MRI-Gerätes geprüft werden. Wenn nicht nur der Abstand der Stufen zueinander, also ihr "Kettenmaß" bekannt ist, sondern auch noch der Abstand zu einem Bezugspunkt des Phantoms, so kann sogar die absolute Genauigkeit der Positionsistwerterfassung durch das MRI-Gerät überprüft werden.

Eine weitere Nutzungsmöglichkeit eines so genau vermessenen Phantoms ist das Training des genauen Positionierens ohne die vorherige - teure - Vermessung in einem MRI-Gerät.

Als ein sinnvolles Verfahren zum Training des exakten Treffens einer mit dem MRI aufgefundenen Anomalie in lebendem Gewebe schlägt die Erfindung vor, dass im ersten Schritt die Grundplatte mit dem darauf befestigten Phantom unterhalb einer MRI-Spule befestigt wird. Im zweiten Schritt wird die Position der im Phantom enthaltenen Zielkörper mit einem MRI-Gerät vermessen. Dann wird im dritten Schritt die Grundplatte mit dem Phantom von der MRI-Spule und dem MRI-Gerät getrennt und im vierten Schritt das medizinische Werkzeug nur auf der Basis der im zweiten Schritt ermittelten Positionsdaten mit seiner Spitze in einen Zielkörper geführt. Als fünfter und letzter Schritt wird das Ergebnis jeweils visuell durch die Wand des Phantoms und durch das Gel hindurch überprüft.

Um eine Werkzeughalterung zu justieren schlägt die Erfindung vor, dass die ersten drei Schritte des zuvor genannten Verfahrens wiederholt werden. Im vierten Schritt wird die Spitze des Werkzeuges mittels der Werkzeugführung unter visueller Kontrolle in einen Zielkörper geführt. Im fünften Schritt wird dann der jeweilige Bezugspunkt und der jeweilige Verfahrweg aller Bewegungsachsen des Werkzeughalters erfasst und zu denjenigen Positionsdaten in rechnerische Beziehung gesetzt, die mit dem MRI-Gerät im zweiten Schritt ermittelt worden sind. Bei künftigen Untersuchungen an real existierendem, lebenden Gewebe kann die auf diese Weise ermittelte rechnerische Beziehung für die Umsetzung der mit dem MRI-Gerät ermittelten Positionsdaten in Positionssollwerte für die Bewegungsachsen des Werkzeughalters genutzt werden.

Um die manuelle Werkzeugführung für das Treffen einer mit dem MRI aufgefundenen Anomalie in lebendem Gewebe schon vorweg an einem Phantom zu üben, empfiehlt die Erfindung einen Zyklus, dessen erste vier Schritte dem zuvor erläuterten Ablauf entsprechen. Im fünften Schritt werden der jeweilige Quadrant zwischen den nächstliegenden vertikalen und horizontalen Andrückschienen des Gewebeandrückrahmens sowie die Anstellwinkel des Werkzeugs in zwei Ebenen erfasst und zu denjenigen Positionsdaten in rechnerische Beziehung gesetzt, die mit dem MRI-Gerät schon zuvor im zweiten Schritt ermittelt worden sind. Bei künftigen Untersuchungen an real existierendem, lebenden Gewebe können die auf diese Weise ermittelten, rechnerischen Beziehungen für die Umsetzung der mit dem MRI-Gerät ermittelten Positionsdaten in eine korrekte manuelle Führung des medizinischen Werkzeugs sinnvoll genutzt werden.

Im Folgenden sollen weitere Einzelheiten und Merkmale der Erfindung anhand von zwei Beispielen näher erläutert werden. Diese sollen die Erfindung jedoch nicht einschränken, sondern nur erläutern. Es zeigt in schematischer Darstellung:
- Fig. 1: Schrägbild eines MRI Trainingsgerätes mit Geräteandruckrahmen und Phantom von der Werkzeugseite her
- Fig. 2: MRI-Trainingsgerät wie Figur 1, jedoch Seitenansicht
- Figur 3: Horizontalschnitt durch ein MRI-Trainingsgerät wie Figur 1 und 2, jedoch mit einem anderen Phantom mit eingesetztem, treppenartigen Phantomkörper

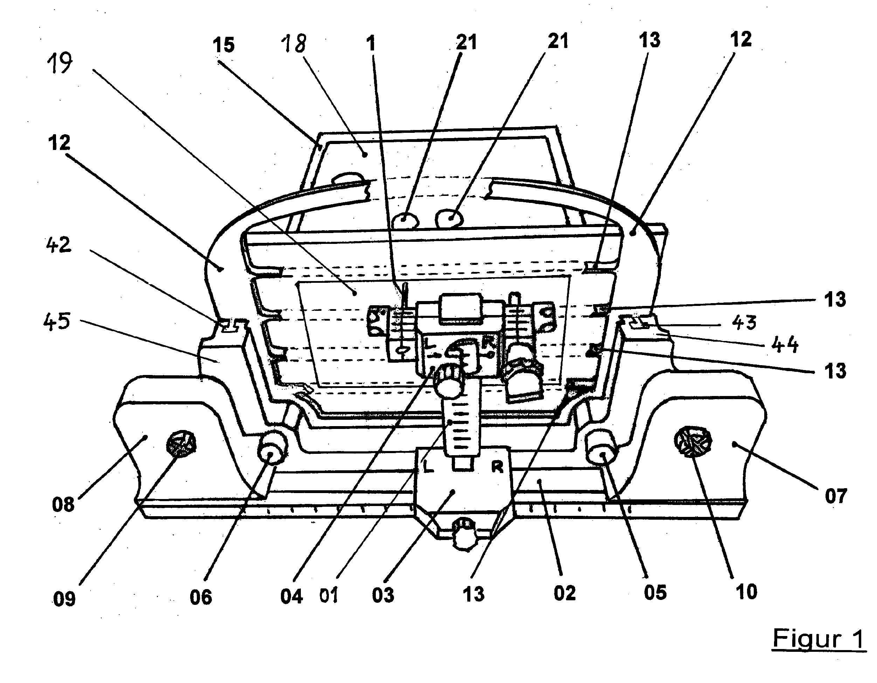

In **Figur 1** ist perspektivisch die Ansicht der Werkzeugseite eines MRI-Trainingsgerätes dargestellt. In Bildmitte ist der Gewebeandrückrähmen 12 zu sehen, der eine große Öffnung aufweist, durch welche mehrere horizontale Andruckschienen 13 verlaufen, die zum größten Teil zeichnerisch aufgebrochen sind und den Blick auf das Phantom 15 frei, das anstelle des menschlichen Gewebes an die Rückseite des Gewebeandrückrahmens 12 angesetzt ist.

In Figur nicht gezeigt wird, dass das Phantom 15 beim normalen Betrieb des MRI-Trainingsgerätes in einem MRI-Gerät ausgebaut wird und die übrige, gezeichnete Anordnung direkt unterhalb einer MRI-Spule des MRI-Gerätes eingesetzt wird. Dann können z.B. durch die MRI-Spule hindurchhängende, weibliche Brüste für eine Diagnose von Gewebeanomalien in eine genau vermessbare Position gebracht werden, indem der Andrückrahmen 12 mittels der beiden Gewindespindeln 05 und 06 soweit an die Brust herangefahren wird, bis er vollflächig darauf aufliegt, wodurch die Brust in eine exakt definierte Form und Position gebracht wird. Dazu verfügt der Gewebeandrückrahmen 12 in seiner großen Öffnung über zahlreiche Andrückschienen 13.

Dann kann am MRI-Gerät nach Anomalien wie z.B. Krebsgeschwulsten gesucht werden und die Position entdeckter Geschwulste genau vermessen werden. Solchen Krebsgeschwulsten entsprechen im Phantom 15 die Zielkörper 21.

Die Positionsdaten der entdeckten Geschwulste b.z.w. der Zielkörper werden vom MRI-Gerät abgelesen. Dorthin muss dann die Spitze des medizinischen Werkzeugs 1 geführt werden.

Im einfachsten - in Figur 1 nicht gezeigten, aber gut nachvollziehbaren - Fall des manuellen Einführens vom medizinischen Werkzeug 1 in das lebende Gewebe können die Andrückschienen 13 auch als Auflage oder zumindest als Orientierungshilfe dienen.

In den meisten Fällen ist das medizinische Werkzeug 1 eine Hohlnadel, die in das menschliche Gewebe gestochen wird. Dieser Einstich muss außerhalb des MRI-Gerätes und daher ohne Kontrollmöglichkeit durchgeführt werden. Nach jedem Einstich muss also der Patient zurück in das MRI-Gerät gefahren werden, um zu kontrollieren, ob Richtung und Eindringtiefe des medizinischen Werkzeuges 1 korrekt sind.

Da eventuelle Fehler bei dieser Positionierungsaufgabe für den Patienten höchst schmerzhaft sind, kann der Arzt das korrekte Einstechen an dem erfindungsgemäßen MRI-Trainingsgerät üben, was in Figur 1 gezeigt wird. Dazu wird anstelle des menschlichen Gewebes das Phantom 15 eingesetzt.

In Figur 1 ist zu erkennen, dass das Phantom 15 in seinem Inneren einige kleine Zielkörper 21 enthält. Sie sind von dem Gel 18 umgeben, das im MRI nicht oder fast nicht sichtbar ist. Ebenso sind auch im MRI-Gerät die Wände des Phantoms 15 unsichtbar, sodass das MRI-Gerät die Zielkörper 21 erkennen und ihre Position vermessen kann. Auch optisch sind die Wände und das Gel 18 durchsichtig, so dass das Ergebnis der Zielsuche stets optisch kontrolliert werden kann.

In Figur 1 ist auch nachvollziehbar, wie mit der Trainingseinrichtung auch das Gefühl des Mediziners für das Einbringen des medizinischen Werkzeuges wie z.B. das Einstechen mit einem nadelförmigen medizinischen Werkzeug 1 in das Phantom 15 geübt werden kann. Dazu ist an der Vorderseite des Phantoms 15 eine Membran 19 angebracht. Sie verschließt die zum Gewebeandrückrahmen 12 weisende Öffnung des Phantoms 15 und hält dadurch das im Inneren des Phantoms 15 befindliche Gel 18 zurück. Sie ist zwar für das medizinische Werkzeug 1 durchdringbar, jedoch mit einem erheblich höheren Widerstand als das Gel 18. In Figur ist gezeichnet, wie ein medizinisches Werkzeug 1 in die Membran 19 eintritt. Nicht wiedergegeben ist, dass sich dabei die Membran 19 zuerst um das Werkzeug herum etwas verformt, bis sie etwas eingeschnitten wird und das Werkzeug 1 durch den einschnitt hindurch eintritt. Die elastische Membran verhindert, dass neben der Einstichstelle Gel 18 heraustritt.

Das Ausführungsbeispiel in Figur 1 zeigt eine mehrfach verstellbare Werkzeughalterung für das medizinische Werkzeug 1. Sie ruht auf der Werkzeugträgerplatte 02, die an beiden Enden an je einem Steg 07 und 08 befestigt ist. Diese Stege 07, 08 sind über Zapfen 09, 10 mit den Befestigungsblöcken 44 und 45 verbunden. Diese Befestigungsblöcke 44,45 tragen wiederum den Gewebeandrückrahmen 12.

Im gezeigten Ausführungsbeispiel ist auf der Werkzeugträgerplatte 02 ein Horizontalschlitten 03 nach links (L) und nach rechts (R) verschiebbar. In Figur 1 ist gut zu erkennen, dass der Horizontalschlitten 03 an einer Markierung an der Vorderseite der Werkzeugträgerplatte 02 entlangfährt. Sein Positionsistwert ist durch eine Skala ablesbar. Durch die in der Mitte des Horizontalschlittens 03 erkennbare Rändelschraube kann die Position des Horizontalschlittens 03 fixiert werden.

In ähnlicher Weise ist auf dem Horizontalschlittens 03 der Vertikalschlitten 01 errichtet, der die Bewegung des medizinischen Werkzeuges in vertikaler Richtung übernimmt.

Zusätzlich kann in dem in Figur 1 gezeigten Ausführungsbeispiel noch die Neigung des medizinischen Werkzeuges durch eine nicht näher bezeichnete, am rechten Rande des Tiefenschlittens 04 angeordnete Schwenkachse geändert werden.

Oben auf dem Vertikalschlitten 01 ist als weiterer Schlitten der Tiefenschlitten 04 positioniert, mit dem das medizinische Werkzeug 1 auf die Membran 19 geschoben werden kann, bis die Spannung der Membran 19 so groß wird, dass sie das medizinische Werkzeug 1 durchlässt. Es bewegt sich im weiteren Verlauf dann mit relativ geringem Widerstand durch das Gel 18 hindurch bis es (hoffentlich) auf einen Zielkörper 21 stößt.

Dadurch befindet sich die Werkzeughalterung stets in einem eindeutigen geometrischen Verhältnis zu den Zielkörpern 21.

In Figur 1 ist sehr gut nachvollziehbar, dass ein wesentlicher Effekt des MRI-Trainingsgerätes ist, dass das Werkzeug 1 beim Training nicht so wie in der Praxis am lebenden Gewebe in ein absolut undurchsichtiges Körperteil eingeschoben wird, sondern dass über durchsichtige Wände des Phantoms 15 und dank der Durchsichtigkeit des Gels 18, auch optisch genau verfolgt werden kann, an welcher Stelle die Spitze des medizinischen Werkzeuges (Hohlnadel) sich derzeit gerade befindet und welchen Weg sie noch zurücklegen muss, um einen Zielkörper 21 erfolgreich zu treffen.

In Figur 1 ist in den beiden Befestigungsblöcken 44 und 45 als eine mögliche Ausführungsvariante eingezeichnet, dass sie jeweils eine T-förmige Führung 42, 43 aufweisen, in die je ein dazu komplementäres Teil auf dem Gewebeandrückrahmen 12 eingreift. Dadurch kann der Gewebeandrückrahmen 12 von oben her in die Befestigungsblöcke 45 und 44 eingeschoben werden und ist dort auch über eine längere Betriebszeit hinweg dort gut gesichert.

In Figur 1 ist nachvollziehbar, dass anstelle des Gewebeandrückrahmens 12 auch das Phantom 15 direkt mit den Befestigungsblöcken 44, 45 verbunden werden kann, wenn es ebenfalls an seiner Vorderseite Befestigungselemente aufweist, die in die Führungen 42 und 43 eingeschoben werden können.

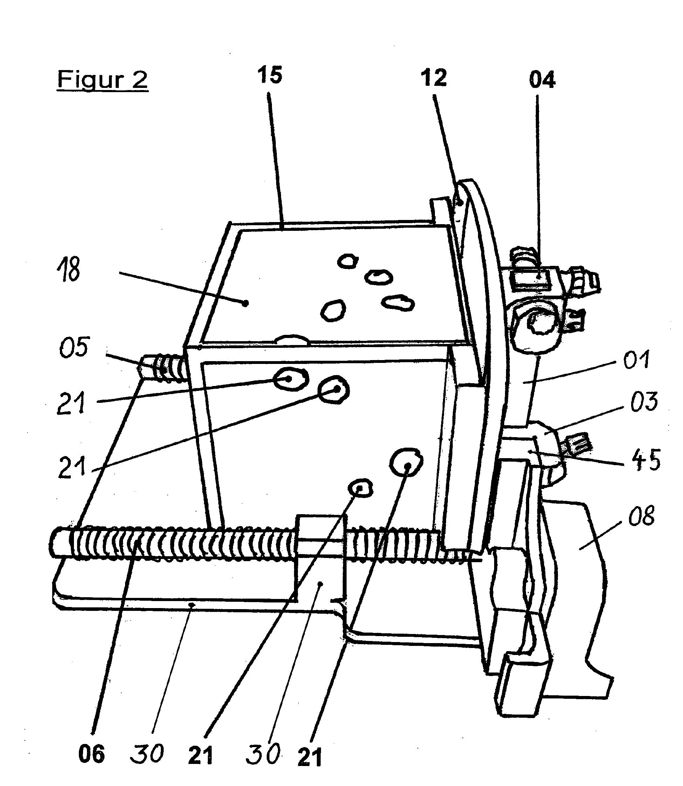

In **Figur 2** ist die gleiche Anordnung wie in Figur 1 von der Seite her perspektivisch dargestellt. Gut zu erkennen ist, dass in diesem Ausführungsbeispiel das Phantom 15 aus einem quaderförmigen Gehäuse besteht, das an seinen Kanten durch einen Rahmen gebildet werden und dessen Flächen jeweils durchsichtig sind, sodass sie den Blick auf Zielkörper 21 freigeben. Diese befinden sich in einer nicht näher definierten Position innerhalb des ebenfalls durchsichtigen Gels 18, das den Innenraum des Phantoms ausfüllt.

In Figur 2 wird deutlich, dass das quaderförmige Phantom 15 mit seiner Vorderfront an der Rückseite des Gewebeandrückrahmens 12 befestigt ist.

Figur 2 macht deutlich, dass die gesamte Baugruppe aus Phantom 15 und Gewebeandrückrahmen 12 und den Befestigungsblöcken 44 und 45 sowie die daran befestigten Stege 07 und 08 mitsamt dem daran befestigten Werkzeughalter über die Befestigungsblöcke 45 und 44 an einem Ende jeweils einer Gewindespindel 05 und 06 befestigt sind. Durch Verdrehen der Gewindespindeln kann die gesamte Baugruppe gegenüber der Grundplatte 30 verschoben werden.

Diese Verschiebung ist für den Trainingsbetrieb nicht relevant, ist jedoch das entscheidende Funktionsmerkmal für die Nutzung des MRI-Trainingsgerätes mit abgenommenem Phantom 15. Dann wird durch Verdrehen der beiden Gewindespindeln 05 und 06 der Gewebeandrückrahmen an das zu untersuchende lebende Gewebe herangedrückt.

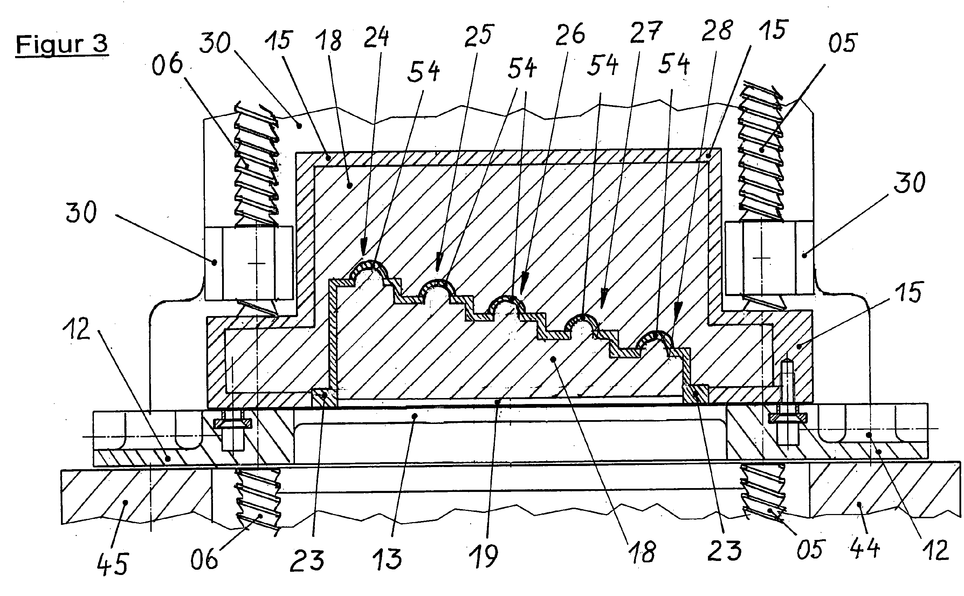

In **Figur 3** ist ein Horizontalschnitt durch die gleiche mechanische Anordnung wie in den Figuren 1 und 2 dargestellt, jedoch mit einem etwas veränderten Phantom 15, der in seinem Inneren direkt gegenüber der Membran 19 einen Phantomkörper 23 trägt. Dieser Phantomkörper 23 weist die treppenartigen Stufen 24 bis 28 auf, die im gezeichneten Ausführungsbeispiel jeweils um den gleichen Betrag von ihren beiden Nachbarn entfernt sind. Auf jeder der fünf Stufen 24 bis 28 ist je eine Einsenkung 54 angebracht, im gezeichneten Ausführungsbeispiel halbkugelförmig. Darin wird ein - hier nicht eingezeichneter - Zielkörper 21 platziert.

Wenn die genauen Abstände der Stufen 24 bis 28 von der Membran 19 bekannt sind und auch die anderen Koordinaten der Einsenkung 54, dann ist in Figur 3 nachvollziehbar, wie mit einem derart konfiguriertes MRI-Trainingsgerät auch ohne die Inanspruchnahme der kostenträchtigen vorherigen Vermessung der Zielkörper 21 durch ein MRI-Gerät das zielgenaue Stechen des medizinischen Werkzeuges geübt werden kann.

Anstelle der Positionsistwerte aus dem MRI-Gerät wird mit den bekannten Positionen der Einsenkungen 54 gearbeitet. Das Ergebnis kann in einfacher Weise optisch kontrolliert werden.

Bei Betrachtung von Figur 3 ist gut nachvollziehbar, dass die hier in jeweils gleicher Höhe eingezeichneten Einsenkungen 54 in verschiedenen Positionen vertikal zu Schnittebene angeordnet werden können, was das Training interessanter und realitätsnäher macht. In Figur 3 sind nur der guten Übersicht halber alle fünf Einsenkungen 54 auf gleicher Höhe eingezeichnet.

### Bezugszeichenliste

- 01: Vertikalschlitten
- 02: Werkzeugträgerplatte
- 03: Horizontalschlitten
- 04: Tiefenschlitten
- 05: Gewindespindel, rechts für Befestigungsblock 44
- 06: Gewindespindel, links für Befestigungsblock 45
- 07: Steg, rechts; an Befestigungsblock 44 mit Zapfen 10 befestigt
- 08: Steg, links; an Befestigungsblock 45 mit Zapfen 9 befestigt
- 09, 10: Zapfen, Verbindung der Stege 07,08 mit den Befestigungsblöcken 44,45
- 1: medizinisches Werkzeug, greift durch Gewebeandrückrahmen 12 hindurch in lebendes Gewebe oder in das Phantom 15 ein
- 12: Gewebeandrückrahmen, an lebendes Gewebe andrückbar
- 13: Andrückschiene, in Öffnung im Gewebeandrückrahmen 12
- 15: Phantom, alternativ zum lebenden Gewebe an Befestigungsblock 44,45 oder an Gewebeandrückrahmen 12 ansetzbar
- 18: Gel, im Innenraum des Phantoms 15
- 19: Membran, verschließt die zum Gewebeandrückrahmen 12 weisende Öffnung des Phantoms 15
- 21: Zielkörper, im MRI sichtbar
- 23: Phantomkörper im Phantom 15
- 24-28: treppenartige Stufe des Phantomkörpers 23
- 30: Grundplatte, trägt Befestigungen für Gewindespindeln 05,06
- 42: Führung, links, zum Befestigen des Phantoms 15 oder des Gewebeandrückrahmens 12
- 43: Führung, wie 42, jedoch rechts
- 44: Befestigungsblock, rechts, trägt Phantom 15 oder Gewebeandrückrahmen 12
- 45: Befestigungsblock, wie 44, jedoch links,
- 54: Einsenkung in Stufen 24-28 für Zielkörper 21

## Patentansprüche

1. Verfahren zum Training des Treffens einer mit dem MRI aufgefundenen Anomalie im lebenden Gewebe mit einem medizinischen Werkzeug mit folgenden Schritten:
- im ersten Schritt wird mit Hilfe eines von einer Grundplatte (30) getragenen, gegenüber dem Gewebe linear verschiebbaren Befestigungsblocks (44, 45) ein Phantom lösbar befestigt, wobei Grundplatte (30) und Befestigungsblock (44, 45) im MRI unsichtbar sind und
- im zweiten Schritt wird die Grundplatte (30) mit dem darauf befestigten Phantom (15) unterhalb einer MRI-Spule für das zu untersuchende lebende Gewebe befestigt und
- im dritten Schritt wird die Position der im MRI unsichtbaren Phantom (15) enthaltenen, im MRI bildgebenden Zielkörper (21) mit einem MRI-Gerät vermessen und
- im vierten Schritt wird die Grundplatte (30) mit dem Phantom (15) vor der MRI-Spule und dem MRI-Gerät getrennt und
- im fünften Schritt wird das medizinische Werkzeug (1) nur auf der Basis der im zweiten Schritt ermittelten Positionsdaten mit seiner Spitze in einen Zielkörper (21) geführt und
- im sechsten Schritt wird die Abweichung der Position der Spitze vom Zielkörper (21) visuell, durch die Wand des Phantoms und durch das Gel (18) hindurch überprüft, wobei Gel und Wände im optischen Bereich durchsichtig sind.

2. Verfahren zur Justage der Werkzeughalterung eines medizinischen Werkzeugs für das Treffen einer mit dem MRI aufgefundenen Anomalie im lebenden Gewebe nach Anspruch 1,
**dadurch gekennzeichnet, dass**
der Ablauf von ersten bis zum dritten Schritt dem Anspruch 1 entspricht und
- im vierten Schritt die Spitze des Werkzeugs (1) mittels der Werkzeugführung unter visueller Kontrolle in einen Zielkörper (21) geführt wird, wobei die visuelle Kontrolle durch optisch durchsichtige Wände und optisch durchsichtiges Gel (18) des Phantoms (15) erfolgt, und
- im fünften Schritt der jeweilige Bezugspunkt und der jeweilige Verfahrweg aller Bewegungsachsen des Werkzeughalters erfasst und zu denjenigen Positionsdaten in rechnerische Beziehung gesetzt werden, die mit dem MRI-Gerät im zweiten Schritt ermittelt worden sind und
- bei künftigen Untersuchungen an real existierendem, lebenden Gewebe die auf diese Weise ermittelte rechnerische Beziehung für die Umsetzung der mit dem MRI-Gerät ermittelten Positionsdaten in Positionssollwerte für die Bewegungsachsen des Werkzeughalters genutzt werden.

3. Verfahren zum Training der manuellen Werkzeugführung eines medizinischen Werkzeugs für das Treffen einer mit dem MRI aufgefundenen Anomalie im lebenden Gewebe nach Anspruch 2, **dadurch gekennzeichnet, dass**
der Ablauf vom ersten bis zum vierten Schritt dem Anspruch 2 entspricht und
- im fünften Schritt der jeweilige Quadrant zwischen den nächstliegenden Andrückschienen des Gewebeandrückrahmens und die Anstellwinkel des Werkzeugs (1) in zwei Ebenen erfasst und zu denjenigen Positionsdaten in rechnerische Beziehung gesetzt werden, die mit dem MRI-Gerät im zweiten Schritt ermittelt worden sind und
- bei künftigen Untersuchungen an real existierendem, lebenden Gewebe die auf diese Weise ermittelte rechnerische Beziehung für die Umsetzung der mit dem MRI-Gerät ermittelten Positionsdaten in die manuelle Führung des medizinischen Werkzeugs genutzt werden.

4. MRI-Trainings- und Justage-Einrichtung für das Positionieren der Spitze eines medizinischen Werkzeugs (1) in lebendem Gewebe, bestehend aus
- einem Phantom (15),
- das mit einem niedrig viskosen, gallertartigen Gel (18) oder einem anderen, dem menschlichen Gewebe in seinen mechanischen Eigenschaften und in seinem Bild im MRI nahe kommenden Material befüllt ist,
- dessen Füllmaterial und Wände im optischen Bereich durchsichtig sind,
- das in seinem Innenraum Zielkörper (21) enthält, die im MRI bildgebend sind und
- dessen übrige Bestandteile im MRI unsichtbar sind,
wobei ein weiterer Bestandteil der Trainingseinrichtung eine Grundplatte (30) ist,
- die unterhalb einer MRI-Spule für das zu untersuchende lebende Gewebe befestigbar ist und
- die wenigstens einen, gegenüber dem Gewebe linear verschiebbaren Befestigungsblock (44,45) trägt, an dem lösbar das Phantom (15) befestigbar ist, wobei
Grundplatte (30) und Befestigungsblock (44, 45), im MRI unsichtbar sind.

5. MRI-Trainings-Einrichtung nach Anspruch 4, **dadurch gekennzeichnet, dass** das Phantom (15) ein Behälter ist, dessen zur Öffnung des Gewebeandrückrahmens (12) weisende Fläche aus einer elastischen, hautartigen Membran (19) besteht, die von dem medizinischen Werkzeug (1) durchdringbar ist.

6. MRI-Trainings-Einrichtung nach Anspruch 4 **dadurch gekennzeichnet, dass** das Gel (18) eine Mischung aus 7 - 20 % Substanzatine, 80 - 93 % Wasser, Konservierungsstoffen, Quervernetzer und Antibiotika ist..

7. MRI-Trainings-Einrichtung nach einem der vorhergehenden Ansprüche 4 - 6, **dadurch gekennzeichnet, dass** auf der Grundplatte (30) zwei Befestigungsblöcke (44, 45) beabstandet zueinander angeordnet sind,
- die mittels je einer Gewindespindel (05, 06) in Richtung des lebenden Gewebes verschiebbar sind und
- an denen mittels je einer Führung (42, 43) das Phantom (15) oder der Gewebeandrückrahmen (12) befestigbar sind.

8. MRI-Trainings-Einrichtung nach einem der vorhergehenden Ansprüche 4 - 7, **dadurch gekennzeichnet, dass** das Phantom (15) mit dem Gewebeandrückrahmen (12) verbunden ist, der in die Führungen (42, 43) der Befestigungsblöcke (44, 45) eingeschoben ist.

9. MRI-Trainings-Einrichtung nach einem der vorhergehenden Ansprüche 4 - 8, **dadurch gekennzeichnet, dass** die Grundplatte (30) eine, in wenigstens drei räumlichen Freiheitsgraden verstellbare Werkzeughalterung für das medizinische Werkzeug (1) trägt.

10. MRI-Trainings-Einrichtung nach Anspruch 9, **dadurch gekennzeichnet, dass** die Werkzeughalterung über mehrere
- orthogonal zueinander ausgerichtete Schlitten und/oder
- Schwenkvorrichtungen
verstellbar ist.

11. MRI-Trainings-Einrichtung nach Anspruch 10, **dadurch gekennzeichnet, dass** die Schlitten und/oder die Schwenkvorrichtungen
- manuell verstellbar und durch je eine Klemmschraube fixierbar sind oder
- mit je einem Motor bewegbar und
- über eine mechanische Skala der jeweilige Positionsistwert ablesbar ist oder
- je ein elektronischer Positionsistwert angebaut ist.

12. MRI-Trainings-Einrichtung nach Anspruch 11, **dadurch gekennzeichnet, dass**
- die Motoren auf Tastendruck bewegbar sind und/oder
- die Positionsistwertgeber mit einer Positionsistwertanzeige verbunden sind und/oder
- Motoren und Positionsistwertgeber mit einer Steuerung verbunden sind.

13. MRI-Trainings-Einrichtung nach einem der vorhergehenden Ansprüche 4 - 12, **dadurch gekennzeichnet, dass** im Innenraum des Phantoms (15) ein Phantomkörper (23) mit treppenartigen Stufen (24, 25, 26, 27) eingebracht ist, wobei in jeder Stufe eine Einsenkung (54) eingeformt ist, die je einen Zielkörper (21) aufnimmt.

14. MRI-Trainings-Einrichtung nach Anspruch 13, **dadurch gekennzeichnet, dass** die Einsenkung (54) kugelsegmentförmig ist.

## Claims

1. Method for training the locating of an anomaly discovered by means of MRI in living tissue with a medical instrument according to one of the preceding claims, comprising the following steps
- In the first step, with the aid of a fastening block (44, 45), which is supported by a base plate (30) and is linearly displaceable with respect to the tissue, a phantom is detachably fastened, the base plate (30) and fastening block (44, 45) being invisible in MRI, and
- In the second step, the base plate (30), with the phantom (15) fastened thereon, is fastened below an MRI coil for the living tissue to be examined and
- In the third step, the position of the MRI-imaging target body (21), which is contained in the MRI-invisible phantom (15) is measured with an MRI device, and
- In the fourth step, the base plate (30), with the phantom (15) is separated from the MRI coil and the MRI device, and
- in the fifth step, the medical instrument (1) is guided with its tip into a target body (21) based solely on the position data determined in the second step and
- in the sixth step, the deviation of the position of the tip of the target body (21) is visually checked through the wall of the phantom and through the gel (18), the gel and walls being transparent in the optical range.

2. Method for adjusting the instrument holder of a medical instrument for locating an anomaly in living tissue identified by MRI according to Claim 1,
**characterised in that**
the procedure from the first to the third step corresponds to Claim1 and
- in the fourth step, the tip of the instrument (1) is guided by means of the instrument guide, under visual control, in a target body (21), visual control taking place via optically transparent walls and optically transparent gel (18) of the phantom (15)
and
- In the fifth step, the reference point in each case and the travel path in each case covers all the axes of movement of the instrument holder, and is set in computational relationship to the position data that have been determined in the second step by means of the MRI unit
and
- for future examinations on actually existing living tissue, the theoretical relationship for implementing the position data obtained by means of the MRI unit are used in position setpoint values for the axes of movement of the instrument holder.

3. Method for training the manual instrument guidance of a medical instrument for locating an anomaly in living tissue identified by MRI according to Claim 2, **characterised in that**
the procedure from the first to the fourth step corresponds to Claim 2, and
- In the fifth step, the quadrant in each case between the nearest pressure rails of the tissue pressure frame and the setting angle of the instrument (1) is registered in two planes is and is set in computational relationship to those position data that have been determined in the second step by means of the MRI unit
and
- for future examinations on actually existing living tissue, the computational relationship determined in this manner is used for implementing the position data obtained by means of the MRI unit in the manual guidance of the medical instrument.

4. MRI training and adjustment device for positioning the tip of a medical instrument (1) in living tissue, consisting of
- a phantom (15),
- which is filled with a low-viscosity gelatinous gel (18) or another material that is similar to human tissue in its mechanical properties and in its MRI image,
- of which the filling material and walls are transparent in the optical range,
- which contains target bodies (21) in its interior, which are imaging in MRI, and
- of which the other components are invisible in MRI,
another component of the training device being a base plate (30),
- that can be fastened below an MRI coil for the living tissue to be examined and
- which bears at least one fastening block (44, 45) that is linearly displaceable with respect to the tissue and on which the phantom (15) can be detachably fastened, wherein
the base plate (30) and fastening block (44, 45) are invisible in MRI.

5. MRI training device according to Claim 4, **characterized in that** the phantom (15) is a container, of which that surface that faces the opening of the tissue pressure frame (12) comprises an elastic skin-like membrane (19) which can be penetrated by the medical instrument (1).

6. MRI training device according to Claim 4, **characterized in that** the gel (18) is a mixture of 7-20% gelatine, 80-93% water, preservatives, crosslinkers and antibiotics.

7. MRI training device according to one of the preceding claims 4-6, **characterized in that**, on the base plate (30) there are arranged two fastening blocks (44, 45) that are spaced from one another
- and are displaceable in the direction of the living tissue by means of a threaded spindle (05, 06) in each case and
- on which the phantom (15) or the tissue pressure frame (12) can be fastened by means of a guide (42, 43) in each case.

8. MRI training device according to one of the preceding claims 4-7, **characterized in that** the phantom (15) is connected to the tissue pressure frame (12), which is inserted into the guides (42, 43) of the fastening blocks (44, 45).

9. MRI training device according to one of the preceding claims 4-8, **characterized in that** the base plate (30) bears an instrument holder (1), which is adjustable in at least three spatial degrees of freedom, for the medical instrument (1).

10. MRI training device according to Claim 9, **characterized in that** the instrument holder is adjustable by means of a plurality of
- slides that are oriented orthogonally with respect to one another and/or
- pivoting devices.

11. MRI training device according to Claim 10, **characterised in that** the slides and/or the pivoting devices
- are manually adjustable and can be fixed by means of a clamping screw in each case or
- can be moved by means of a motor and
- the respective position actual value can be read from a mechanical scale or
- an electronic position actual-value encoder in each case is mounted.

12. MRI training device according to Claim 11, **characterised in that**
- the motors are movable at the push of a button and/or
- the position actual value encoders are connected to a position actual-value indicator and/or
- motors and position actual value encoders are connected to a control.

13. MRI training device according to one of the preceding claims 4-12, **characterised in that**, in the interior of the phantom (15), a phantom body (23), with step-like stages (24, 25, 26, 27) is introduced, a depression (54) being moulded-in in each stage, which receives a target body (21) in each case.

14. MRI training device according to Claim 13, **characterised in that** the depression (54) is spherical-segment-shaped.

## Revendications

1. Procédé destiné à apprendre la rencontre d'une anomalie détectée par l'IRM dans un tissu vivant avec un outil médical en suivant les étapes suivantes :
- dans une première étape, un fantôme est fixé de façon détachable à l'aide d'un bloc de fixation (44, 45) porté par une plaque de base (30) pouvant être déplacé de façon linéaire par rapport au tissu, sachant que la plaque de base (30) et le bloc de fixation (44, 45) sont invisibles dans l'IRM,
- la plaque de base (30) avec le fantôme (15) fixé dessus étant, dans une deuxième étape, fixée en-dessous d'une bobine IRM pour le tissu vivant devant être examiné,
- la position des corps cibles (21) contenus dans le fantôme (15), invisibles dans l'IRM et produisant une image dans l'IRM étant, dans une troisième étape, mesurée avec un appareil IRM,
- la plaque de base (30) avec le fantôme (15) étant, dans une quatrième étape, séparée avant la bobine IRM et l'appareil IRM,
- l'outil médical (1) étant, dans une cinquième étape, mené seulement sur la base des données de position déterminées dans la seconde étape avec sa pointe dans un corps cible (21),
- l'écart de la position de la pointe par rapport au corps cible (21) étant, dans une sixième étape, vérifié visuellement, à travers la paroi du fantôme et à travers le gel (18), sachant que le gel et les parois sont transparents dans la zone optique.

2. Procédé destiné à ajuster le porte-outil d'un outil médical pour la rencontre d'une anomalie dans le tissu vivant détectée avec l'IRM selon la revendication 1,
**caractérisé par le fait que**
le déroulement correspond aux étapes 1 à 3 selon la revendication 1
- la pointe de l'outil (1) étant, dans une quatrième étape, menée au moyen du guidage d'outil dans un corps cible (21) sous contrôle visuel, sachant que le contrôle visuel a lieu à travers les parois optiquement transparentes et le gel optiquement transparent (18) du fantôme (15),
- le point de référence respectif et la course de déplacement respective de tous les axes de déplacement du porte-outil étant, dans une cinquième étape, enregistrés et mis en relation de calcul avec les données de position qui ont été déterminées avec l'appareil IRM dans la seconde étape,
- la relation de calcul déterminée de cette manière étant, dans le cadre de futurs examens sur des tissus vivants réellement existants, utilisée pour la transposition des données de position enregistrées avec l'appareil IRM en valeurs de consigne de position pour les axes de déplacement du porte-outil.

3. Procédé destiné à apprendre le guidage d'outil manuel d'un outil médical pour la rencontre d'une anomalie détectée avec l'IRM dans un tissu vivant selon la revendication 2,
**caractérisé par le fait que**
le déroulement correspond aux étapes 1 à 4 de la revendication 2,
- le quadrant respectif étant, dans une cinquième étape, enregistré dans deux niveaux entre les rails de pression les plus proches du cadre de pression du tissu et l'angle de réglage de l'outil (1) et mis en relation de calcul par rapport aux données de position qui ont été déterminées avec l'appareil IRM dans la deuxième étape,
- la relation de calcul déterminée de cette manière étant, dans le cas d'examens futurs sur des tissus vivants réellement existants, utilisée pour la transposition des données de position déterminées avec l'appareil IRM dans le guidage manuel de l'outil médical.

4. Dispositif d'apprentissage et d'ajustement IRM destiné à positionner la pointe d'un outil médical (1) dans du tissu vivant, consistant en
- un fantôme (15),
∘ qui est rempli d'un gel (18) gélatineux à basse viscosité ou d'un autre matériau proche du tissu humain au niveau de ses propriétés mécaniques et de son aspect dans l'IRM,
∘ dont le matériau de remplissage et les parois sont transparents au niveau optique,
∘ qui contient dans son espace intérieur des corps cibles (21) qui produisent une image dans l'IRM et
∘ dont les autres composants dans l'IRM sont invisibles,
sachant qu'un autre composant du dispositif d'apprentissage est une plaque de base (30),
- qui peut être fixé en-dessous d'une bobine IRM pour le tissu vivant à examiner et
- qui porte au moins un bloc de fixation (44, 45) pouvant être déplacé linéairement par rapport au tissu, sur lequel le fantôme (15) peut être fixé de façon détachable, sachant que la plaque de base (30) et le bloc de fixation (44, 45) sont invisibles dans l'IRM.

5. Dispositif d'apprentissage et d'ajustement IRM selon la revendication 4, **caractérisé par le fait que** le fantôme (15) est un récipient dont la surface dirigée vers l'ouverture du cadre de pression du tissu (12) consiste en une membrane (19) élastique semblable à la peau qui peut être traversée par l'outil médical (1).

6. Dispositif d'apprentissage IRM selon la revendication 4
**caractérisé par le fait que** le gel (18) est un mélange de 7 à 20% de gelatine, 80 à 93% d'eau, d'agents de conservation, d'agents de réticulation croisée et d'antibiotiques.

7. Dispositif d'apprentissage IRM selon une des revendications précédentes 4 à 6, **caractérisé par le fait que** deux blocs de fixation (44, 45) placés à une certaine distance l'un de l'autre sont disposés sur la plaque de base (30),
- pouvant chacun être déplacé dans le sens du tissu vivant au moyen d'une broche filetée (05, 06) et
- sur chacun desquels le fantôme (15) ou le cadre de pression de tissu (12) peut être fixé au moyen d'un guidage (42, 43).

8. Dispositif d'apprentissage IRM selon une des revendications précédentes 4 à 7, **caractérisé par le fait que** le fantôme (15) est relié au cadre de pression de tissu (12), qui est inséré dans les guidages (42, 43) des blocs de fixation (44, 45).

9. Dispositif d'apprentissage IRM selon une des revendications précédentes 4 à 8, **caractérisé par le fait que** la plaque de base (30) porte un porte-outil destiné à l'outil médical (1) pouvant être réglé dans au moins trois degrés de liberté spatiale.

10. Dispositif d'apprentissage IRM selon la revendication 9, **caractérisé par le fait que** le porte-outil peut être réglé par l'intermédiaire de plusieurs
- chariots orientés dans un sens orthogonal l'un par rapport à l'autre et/ou
- dispositifs de pivotement.

11. Dispositif d'apprentissage IRM selon la revendication 10, **caractérisé par le fait que** les chariots et/ou dispositifs de pivotement
- peuvent être réglés manuellement et peuvent être chacun fixés à l'aide d'une vis de serrage ou bien
- peuvent chacun être déplacé par un moteur et
- dont la valeur de position respective peut être lue par l'intermédiaire d'une échelle mécanique ou
- sur chacun desquels est monté un encodeur de position réelle électronique.

12. Dispositif d'apprentissage IRM selon la revendication 11,
**caractérisé par le fait que**
- les moteurs peuvent être déplacés par simple pression sur une touche et/ou
- les encodeurs de position réelle sont reliés à un affichage de valeurs de position réelles et/ou
- les moteurs et les encodeurs de position réelle sont reliés à une commande.

13. Dispositif d'apprentissage IRM selon une des revendications précédentes 4 à 12, **caractérisé par le fait qu'**un corps fantôme (23) ayant des niveaux en escalier (24, 25, 26, 27) est disposé à l'intérieur du fantôme (15), sachant qu'un renfoncement (54) est formé dans chaque niveau, chacun de ces renfoncements réceptionnant un corps cible (21).

14. Dispositif d'apprentissage IRM selon la revendication 13, **caractérisé par le fait que** le renfoncement (54) a la forme d'un segment de sphère.
